# EUROPEAN PATENT SPECIFICATION

(11) **EP 2 350 629 B1**
(45) Date of publication and mention of the grant of the patent: **18.12.2013**
(21) Application number: 09826755.2
(22) Date of filing: 12.11.2009
(51) Int. Cl.: G01N 27/26, G01N 33/48, A61B 5/15, A61B 5/151, A61B 5/157, A61B 5/1486, A61M 5/172, A61B 5/145

(54) **ELECTROCHEMICAL SENSOR MODULE**
ELEKTROCHEMISCHES SENSORMODUL
MODULE DÉTECTEUR ÉLECTROCHIMIQUE

(30) Priority: 14.11.2008 US 114829 P
(43) Date of publication of application: 03.08.2011
(73) Proprietor: Pepex Biomedical, LLC, Alameda, CA 94502 (US)
(72) Inventor: SAY, James, L., Breckenridge CO 80424 (US)
(74) Representative: Burt, Matthew Thomas
(86) International application number: PCT/US2009/064216
(87) International publication number: WO 2010/056869

(56) References cited:
- WO-A1-00/35340
- US-A1- 2004 236 251
- US-A1- 2006 241 517
- US-A1- 2007 149 897
- US-B2- 6 561 989
- US-B2- 7 299 081

## Description

### Technical Field

The present disclosure relates to sensors for measuring bioanalytes and to methods for making such sensors.

### Background

Electrochemical bio-sensors have been developed for detecting analyte concentrations in a given fluid sample. For example, United States Patent Nos. 5,264,105; 5,356,786; 5,262,035; 5,320,725; and 6,464,849 disclose wired enzyme sensors for detecting analytes, such as lactate or glucose. Wired enzyme sensors have been widely used in blood glucose monitoring systems adapted for home use by diabetics to allow blood glucose levels to be closely monitored. Other example types of blood glucose monitoring systems are disclosed by United States Patent Nos. 5,575,403; 6,379,317; and 6,893,545.

WO 00/35340 A1 (Pipex Biomedical LLC) published 22 June 2000 discloses a sensor assembly including a catheter having a catheter sheath adapted for insertion in a patient and a catheter hub connected to the catheter sheath.

### Summary

The present invention provides a sensor module as defined in the appended claims.

A variety of additional aspects will be set forth in the description that follows. The aspects can relate to individual features and to combinations of features. It is to be understood that both the forgoing general description and the following detailed description are exemplary and explanatory only and are not restrictive of the broad concepts upon which the embodiments disclosed herein are based.

### Brief Description of the Drawings

FIG. 1 is a distal, perspective view of an electrochemical sensor module in accordance with the principles of the present disclosure;
FIG. 2 is a proximal, perspective view of the electrochemical sensor module of FIG. 1;
FIG. 3 is a plan view of the electrochemical sensor module of FIG.1 with a piercing member in a retracted orientation and a cover of the sensor module removed to better show interior components of the sensor module;
FIG. 4 is a plan view of the sensor module of FIG. 1 with the skin piercing element in an extended position and the cover removed to better show interior components of the sensor module;
FIG. 5 is a perspective view of the electrochemical sensor module of FIG. 1 with the cover transparent to better show interior components of the module;
FIG. 6 is a perspective view of the electrochemical sensor module of FIG. 1 with a cover removed to better show interior components of the module;
FIG. 7 is a block schematic diagram of an analyte monitoring system in which one or more electrochemical sensor modules shown in FIG. 1 can be mounted in accordance with the principles of the present disclosure;
FIG. 8 is a perspective view of one example analyte monitoring system in accordance with the principles of the present disclosure;
FIG. 9 is a perspective view of components of an analyte monitoring system showing how the sensor module of FIG. 1 can be arranged within a cartridge wheel of the system in accordance with the principles of the present disclosure;
FIG. 10 is a perspective view of components of components of the analyte monitoring system of FIG. 9 showing how the sensor module of FIG. 1 latches to a skin-piercing member driver of the monitoring system;
FIG. 11 is a further view showing how the sensor module of FIG. 1 mounts within the analyte monitoring system of FIG. 9;
FIG. 12 is a perspective view of an exterior portion of a case that encloses the cartridge shown at FIG. 9;
FIG. 13 is a further view of the case and cartridge of FIGS. 9 and 12; and
FIG. 14 is a perspective view of another analyte monitoring system showing how the sensor module of FIG. 1 can be arranged within a cartridge wheel of the system in accordance with the principles of the present disclosure.

### Detailed Description

Reference will now be made in detail to exemplary aspects of the present disclosure which are illustrated in the accompanying drawings. Wherever possible, the same reference numbers will be used throughout the drawings to refer to the same or like parts.

The following definitions are provided for terms used herein:
A "working electrode" is an electrode at which the analyte (or a second compound whose level depends on the level of the analyte) is electrooxidized or electroreduced with or without the agency of an electron transfer agent.
A "reference electrode" is an electrode used in measuring the potential of the working electrode. The reference electrode should have a generally constant electrochemical potential as long as no current flows through it. As used herein, the term "reference electrode" includes pseudo-reference electrodes. In the context of the disclosure, the term "reference electrode" can include reference electrodes which also function as counter electrodes (i.e., a counter/reference electrode).
A "counter electrode" refers to an electrode paired with a working electrode to form an electrochemical cell. In use, electrical current passes through the working and counter electrodes. The electrical current passing through the counter electrode is equal in magnitude and opposite in sign to the current passing through the working electrode. In the context of the disclosure, the term "counter electrode" can include counter electrodes which also function as reference electrodes (i.e., a counter/reference electrode).
A "counter/reference electrode" is an electrode that functions as both a counter electrode and a reference electrode.
An "electrochemical sensing system" is a system configured to detect the presence and/or measure the level of an analyte in a sample via electrochemical oxidation and reduction reactions on the sensor. These reactions are converted (e.g., transduced) to an electrical signal that can be correlated to an amount, concentration, or level of an analyte in the sample. Further details about electrochemical sensing systems, working electrodes, counter electrodes and reference electrodes can be found at U.S. Patent No. 6,560,471.
"Electrolysis" is the electrooxidation or electroreduction of a compound either directly at an electrode or via one or more electron transfer agents.
An "electron transfer agent" is a compound that carries electrons between the analyte and the working electrode either directly or in cooperation with other electron transfer agents. One example of an electron transfer agent is a redox mediator.
A "sensing layer" is a component of the sensor which includes constituents that facilitate the electrolysis of the analyte. The sensing layer may include constituents such as an electron transfer agent, a catalyst which catalyzes a reaction of the analyte to produce a response at the electrode, or both.

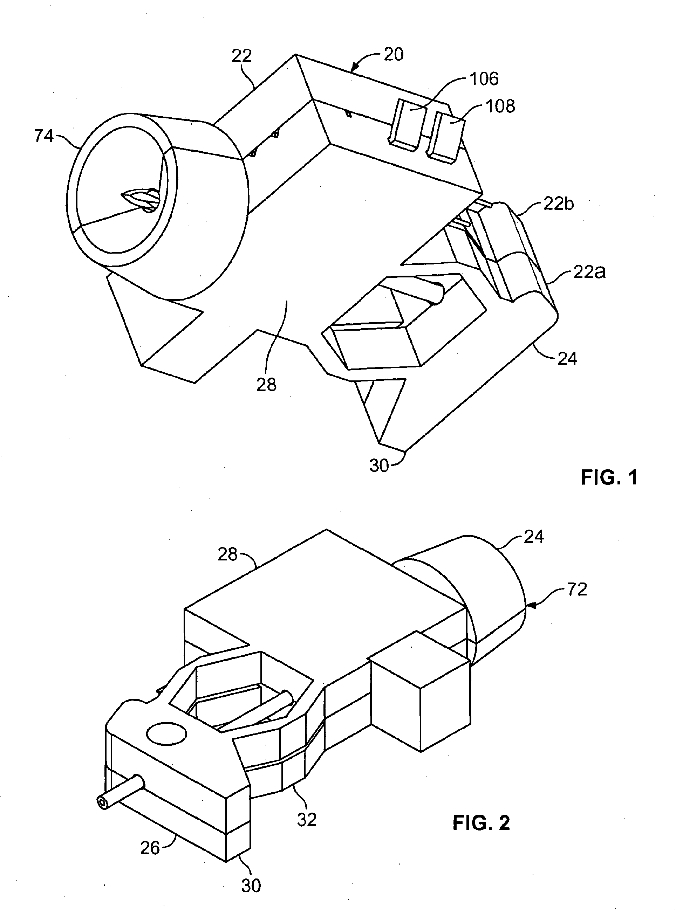

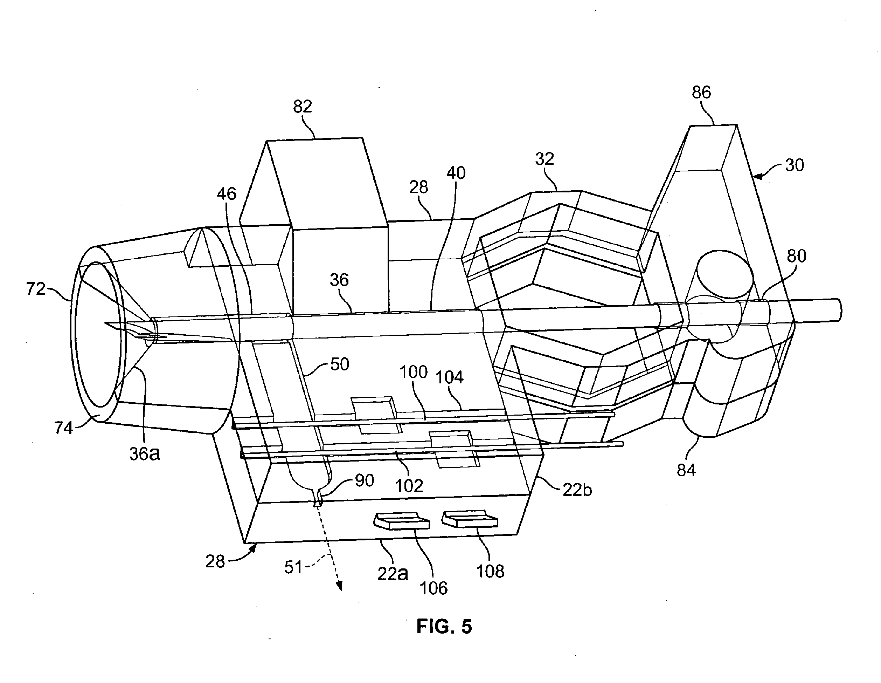

FIGS. 1-6 illustrate a sensor module 20 configured in accordance with the principles of the present disclosure. The sensor module 20 includes a module body 22 having a distal end 24 positioned opposite from a proximal end 26. The module body 22 is preferably constructed of a molded plastic material. For example, in one embodiment, the module body 22 includes a first molded piece 22a secured to a second molded piece 22b at a part line (see FIG. 5). In one embodiment, the parts 22a, 22b are molded using a manufacturing process such as a continuous insert micro-molding process.

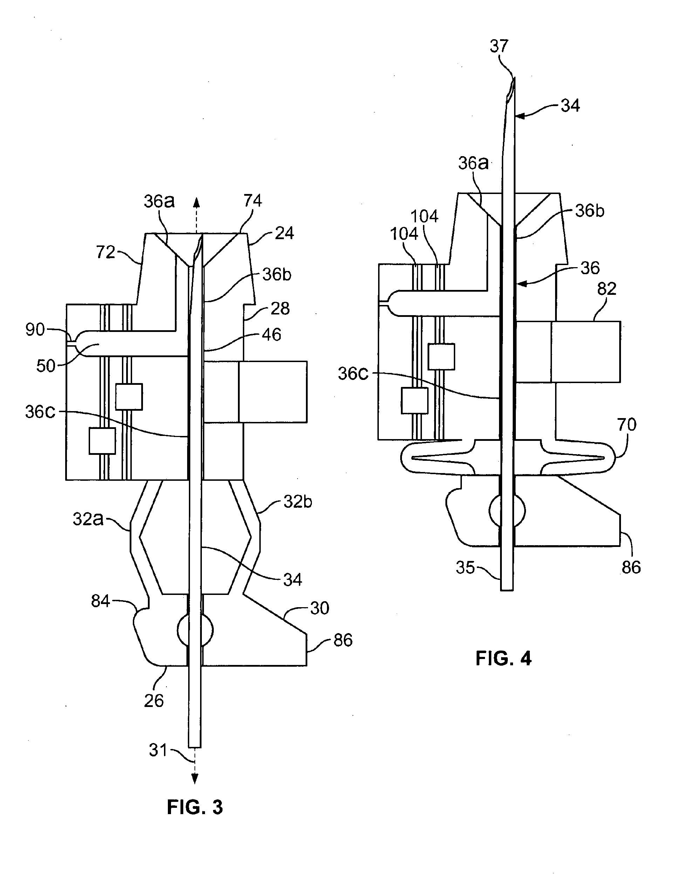

The module body 22 includes an analysis cell housing 28 positioned adjacent the distal end 24 and a skin piercing member anchor 30 positioned adjacent the proximal end 26. A flexible linkage 32 (FIG. 2) mechanically connects the analysis cell housing 28 to the skin piercing member anchor 30. The flexible linkage 32 is configured to allow the analysis cell housing 28 and the skin piercing member anchor 30 to move relative to one another along an axis 31 that extends through the module body 22 from the proximal end 26 to the distal end 24. The analysis cell housing 28 defines an analysis cell 50 (FIGS. 3-6) at which a fluid sample (e.g., a blood sample) can be analyzed using a sensor structure, such as a wired enzyme sensor arrangement, in fluid communication with the analysis cell 50.

The sensor module 20 also includes a skin piercing member 34 (e.g., a cannula, a needle, a lancet, or other structure) aligned along the axis 31 (see FIGS. 3 and 4). The skin piercing member 34 includes a base end 35 positioned opposite from a piercing tip 37. The base end 35 of the skin piercing member 34 is secured to the skin piercing member anchor 30 and the skin piercing member 34 extends distally from the skin piercing member anchor 30 through a passage 36 defined by the analysis cell housing 28. The passage 36 includes a distal end 38 positioned opposite from a proximal end 40. The passage 36 includes a capillary slot 46 (FIGS. 3 and 4) that provides fluid communication between the analysis cell 50 and the distal end 38 of the passage 36.

In use of the sensor module 20, the distal end 24 of the module body 22 is placed against a patient's skin at a sampling location where it is desired to take a fluid (e.g., blood) sample. Once the distal end 24 is in contact with the skin, the skin piercing member anchor 30 can be driven distally along the axis 31 by an actuator (i.e., a driver) that couples to the skin piercing member anchor 30. As the skin piercing member anchor 30 is driven distally, the skin piercing member 34 slides within the passage 36 from a retracted position (see FIG. 3) to an extended position (see FIG. 4) at which the tip 37 of the skin piercing member 34 extends distally beyond the distal end 24 of the module body 22. The distance the skin piercing member 34 extends beyond the distal end 24 of the module body 22 is preferably selected to ensure that a blood sample will be drawn efficiently. The skin piercing member anchor 30 is then pulled back proximally by the actuator causing the tip 37 of the skin piercing member 34 to be retracted back into the passage 36.

Penetration by the skin piercing member 34 into the patient's tissue at a wound site causes a blood sample from the wound site to enter the passage 36 and flow by capillary action through the capillary slot 46 to the analysis cell 50. At the analysis cell 50, an analyte level (e.g., the blood glucose level) in the blood sample is sensed by the wired enzyme sensor arrangement that is typically coupled (e.g., wired) to a controller, such as a microcontroller, a mechanical controller, a software driven controller, a hardware driven controller, a firmware driven controller, etc. The controller can include a microprocessor that interfaces with memory. The controller would typically be integrated into an analyte monitor, such as a glucose monitor, having user interfaces for receiving user input (e.g., buttons and switches) and/or providing user output (e.g., a display for displaying the sensed analyte reading).

The flexible linkage 32 of the module body 22 preferably has a compressible configuration that enables the flexible linkage 32 to compress axially along axis 31 as the skin piercing member anchor 30 moves the skin piercing member 34 from the retracted position to the extended position. As shown in FIGS. 3 and 4, one example flexible linkage 32 includes two linkage members 32a, 32b. Each linkage member 32a, 32b has a first end integrally formed with the skin piercing member anchor 30 and a second end integrally formed with the analysis cell housing 28. Each of the linkage members 32a, 32b includes an intermediate flex or hinge point 70 (e.g., a central hinge point) that enables the linkage member 32a, 32b to flex radially outwardly relative to the axis 31 when the skin piercing member anchor 30 is moved in a distal direction relative to the analysis cell housing 28. The flex or hinge point 70 also enables each linkage member 32a, 32b to flex radially inwardly toward the central axis 31 when the skin piercing member anchor 30 is moved in a proximal direction relative to the analysis cell housing 28. Accordingly, the linkage members 32a, 32b expand radially outwardly from the axis 31 to provide axial shortening of the linkage members 32a, 32b along the axis 31, and contract radially toward the axis 31 to allow axial lengthening of the linkage members 32a, 32b along the axis 31. The flexible linkage 32 also can be referred to as a "dynamic linkage" since it allows for relative movement between the skin piercing member anchor 30 and the analysis cell housing 28.

The passage 36 of the analysis cell housing 28 includes a tapered portion 36a, a sample transport portion 36b, and a skin piercing member guide portion 36c (see FIGS. 3 and 4). The tapered portion 36a has a taper that narrows as the tapered portion 36a extends in a proximal direction through the analysis cell housing 28. As depicted at FIGS. 3-6, the tapered portion 36a of the passage 36 has a truncated, conical shape with a major diameter adjacent the skin engaging surface 74 and a minor diameter adjacent the capillary slot 46. The tapered portion 36a of the passage 36 is provided at a distal tip 72 of the analysis cell housing 28 the module body 22. The distal tip 72 is configured to stabilize an interface between the module body 22 and the patient's skin when a blood sample is being taken. The distal tip 72 includes a circular skin engaging surface 74 concentrically aligned with respect to the axis 31. When a blood sample is being taken, the skin engaging surface 74 is pressed against the patient's skin at the sampling location to stabilize the module body 22 and to facilitate insertion of the skin piercing member 34 into the patient's tissue. The blood sample enters the analysis cell housing 28 through the tapered portion 36a of the passage 36.

The sample transport portion 36b is located between the tapered portion 36a and the skin piercing member guide portion 36c. The sample transport portion 36b has a larger transverse cross-sectional area than the skin piercing member guide portion 36c. The larger cross-section is provided by the capillary slot 46 that extends along the axis 31 from the tapered portion 36a to the analysis cell 50. The capillary slot 46 is sized to provide a capillary space along the skin piercing member 34 for allowing the blood sample to travel by capillary action from the tapered portion 36a of the passage 36 to the analysis cell 50. In this way, the capillary slot 46 provides a direct path for transporting the sample from the interface of the wound site generated by the skin piercing member 34, up along the outer surface of the skin piercing member 34, and into the analysis cell 50. Hydrophilic coatings, selective surface treatments, and/or certain moldable polymers can be used to enhance capillary transport along the sample transport portion 36b of the passage 36.

The skin piercing member guide portion 36c of the passage 36 is preferably sized such that it will provide minimum concentric clearance around the skin piercing member 34. In this way, when the skin piercing member 34 is mounted within the passage 36, the skin piercing member guide portion 36c of the passage 36 allows the skin piercing member 34 to slide within the passage 36 while preventing substantial passage of blood or other interstitial fluid proximally beyond the sample transport portion 36b of the passage 36.

As indicated above, the skin piercing member 34 is secured to the skin piercing member anchor 30. For example, the proximal end of the skin piercing member 34 can be press-fit, adhesively bonded, or otherwise secured within an opening 80 (FIG. 5) defined by the skin piercing member anchor 30 at a location along the axis 31. While the opening 80 is shown as a through-hole, it will be appreciate that the opening 80 could also be a blind hole. Other connection techniques, such as fasteners, snap-fit connections, or other securement arrangements, also could be used to secure the skin piercing member 34 to the piercing member anchor 30.

The analysis cell 50 defined by the analysis cell housing 28 is elongated in a direction that is generally perpendicular relative to the axis 31. The analysis cell 50 has a first end in fluid communication with the sample transport portion 36b of the passage 36 and an opposite, second end at which a vent 90 is defined. The length of the analysis cell 50 is aligned along an axis 51 that is perpendicular relative to the axis 31 defined by the passage 36.

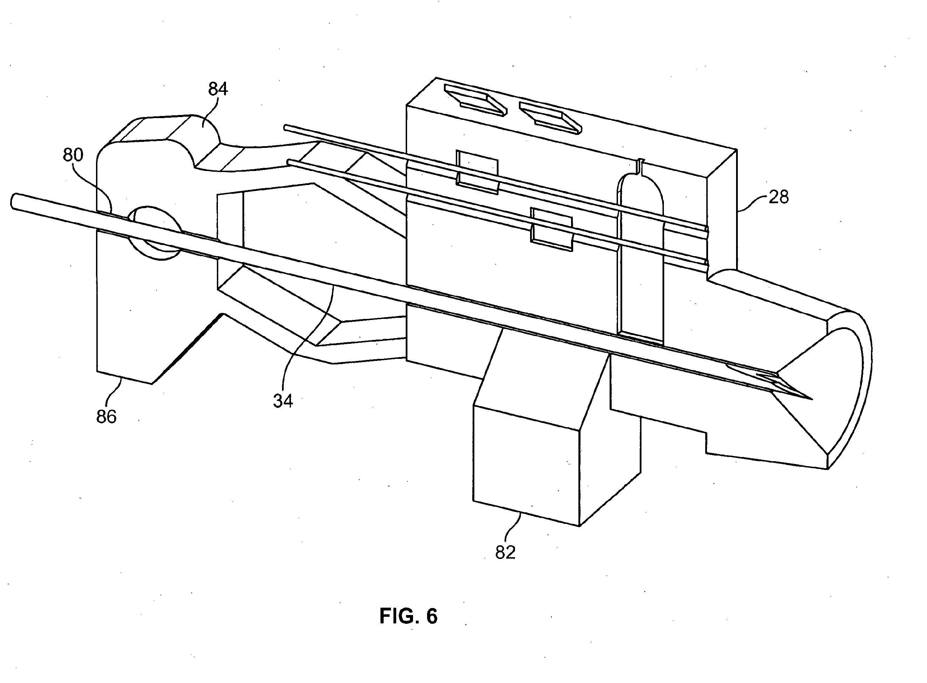

First and second electrodes 100, 102 extend across the analysis cell 50 in a direction generally perpendicular to the axis 51 of the analysis cell 50 (see FIGS. 5 and 6). In one embodiment, the first electrode 100 is in contact with a sensing layer and functions as a working electrode and the second electrode 102 can function as a reference/counter electrode. In other embodiments, separate working, reference and counter electrodes can be provided in fluid communication with the analysis cell 50. The electrodes 100, 102 are preferably threads, fibers, wires, or other elongated members. The analysis cell housing 28 can include electrode mounting structures in which the electrodes 100, 102 are secured. For example, in one embodiment, the electrode mounting structures can include grooves 104 (e.g., V-grooves) that extend through the analysis cell housing 28 in a direction generally perpendicular relative to the axis 51 of the analysis cell 50.

In one embodiment, the working electrode 100 can include an elongated member that is coated or otherwise covered with a sensing layer and the reference/counter electrode 102 can include any elongated member, such as a wire or fiber that is coated or otherwise covered with a layer, such as silver chloride. Preferably, at least a portion of each elongated member is electrically conductive. In certain embodiments, each elongated member can include a metal wire or a glassy carbon fiber. In still other embodiments, each elongated member can have a composite structure and can include a fiber having a dielectric core surrounded by a conductive layer suitable for forming an electrode.

A preferred composite fiber is sold under the name Resistat® by Shakespeare Conductive Fibers LLC. This composite fiber includes a composite nylon, monofilament, conductive thread material made conductive by the suffusion of about a 1 micron layer of carbonized nylon isomer onto a dielectric nylon core material. The Resistat® material is comprised of isomers of nylon to create the basic 2 layer composite thread. However, many other polymers are available for the construction, such as: polyethylene terephthalate, nylon 6, nylon 6,6, cellulose, polypropylene cellulose acetate, polyacrylonitrile and copolymers of polyacrylonitrile for a first component and polymers such as of polyethylene terephthalate, nylon 6, nylon 6,6, cellulose, polypropylene cellulose acetate, polyacrylonitrile and copolymers of polyacrylonitrile as constituents of a second component. Inherently conductive polymers (ICP) such as doped polyanaline or polypyrrole can be incorporated into the conductive layer along with the carbon to complete the formulation. In certain embodiments, the ICP can be used as the electrode surface alone or in conjunction with carbon. The Resistat® fiber is availability in diameters of 0.0635 to 0.4064 millimeters (.0025 to .016 inches), which as suitable for sensor electrodes configured in accordance with the principles of the present disclosure. Example patents disclosing composite fibers suitable for use in practicing sensor modules configured in accordance with the principles of the present disclosure include U.S. Patent Nos. 3,823,035; 4,255,487; 4,545,835 and 4,704,311.

The sensing layers provided at working electrodes of sensor modules configured in accordance with the principles of the present disclosure can include a sensing chemistry, such as a redox compound or mediator. The term redox compound is used herein to mean a compound that can be oxidized or reduced. Example redox compounds include transition metal complexes with organic ligands. Preferred redox compounds/mediators include osmium transition metal complexes with one or more ligands having a nitrogen containing heterocycle such as 2, 2'-bipyridine. The sensing material also can include a redox enzyme. A redox enzyme is an enzyme that catalyzes an oxidation or reduction of an analyte. For example, a glucose oxidase or glucose dehydrogenase can be used when the analyte is glucose. Also, a lactate oxidase or lactate dehydrogenase fills this role when the analyte is lactate. In sensor systems, such as the one being described, these enzymes catalyze the electrolysis of an analyte by transferring electrons between the analyte and the electrode via the redox compound. Further information regarding sensing chemistry can be found at U.S. Patent Nos. 5,264,105; 5,356,786; 5,262,035; and 5,320,725.

In use of the sensor module 20, a fluid sample (e.g., a blood sample) flows through the tapered portion 36a and the sample transport portion 36b of the passage 36 defined in the housing 28 and fills the analysis cell 50. As the analysis cell 50 fills with the fluid sample, the vent 90 allows air within the analysis cell 50 to be displaced by the fluid sample. Once the analysis cell 50 is filled with the fluid sample, a voltage can be applied between the electrodes 100, 102. When the potential is applied, an electrical current will flow through the fluid sample between the electrodes 100, 102. The current is a result of the oxidation or reduction of an analyte, such as glucose, in the volume of fluid sample located within the analysis cell 50. This electrochemical reaction occurs via the electron transfer agent in the sensing layer and an optional electron transfer catalyst/enzyme in the sensing layer. By measuring the current flow generated at a given potential (e.g., with a controller described herein), the concentration of a given analyte (e.g., glucose) in the fluid sample can be determined. Those skilled in the art will recognize that current measurements can be obtained by a variety of techniques including, among other things, coulometric, potentiometric, perometric, voltometric, and other electrochemical techniques.

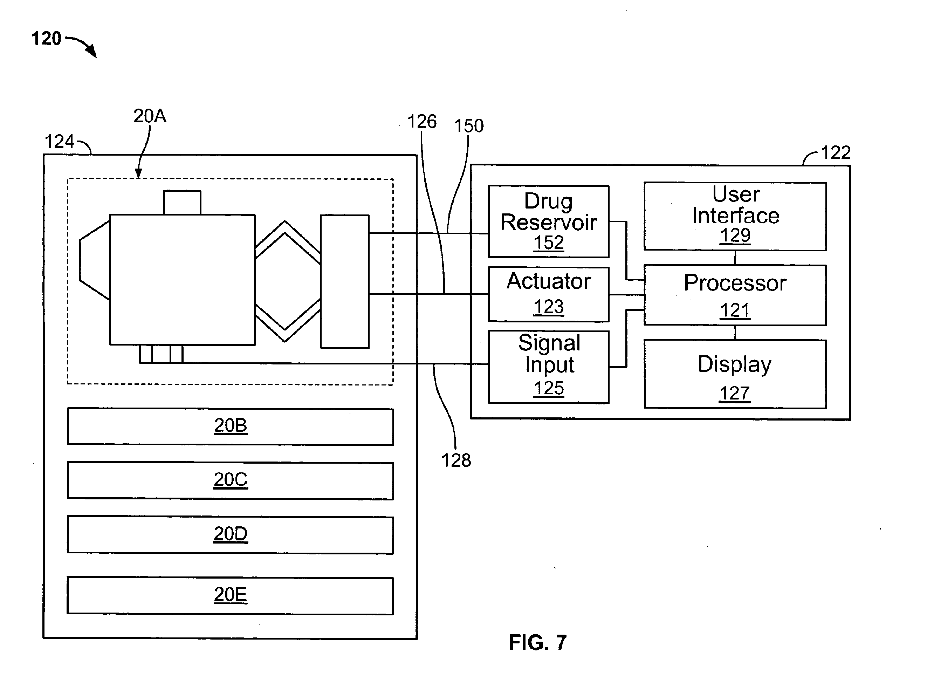

Referring to FIG. 7, it will be appreciated that one or more sensor modules 20 can be incorporated as sub-components into an overall analyte monitoring system 120. The monitoring system 120 includes a controller 122 that couples to a module holder 124. The module holder 124 is configured to hold one or more sensor modules 20. Each sensor module 20 is configured to obtain one or more fluid samples, to measure a concentration level for one or more analytes (e.g., glucose, lactate, etc.), and to generate a signal (e.g., an electrical signal) indicating the concentration level. For example, the module holder 124 shown in FIG. 7 contains five sensor modules 20A-20E. In one embodiment, each sensor module 20 is configured to analyze a single fluid sample. In such an embodiment, the sensor module 20 can be removed from the module holder 124 after one use. In other embodiments, each sensor module 20 can be configured to analyze a greater number of fluid samples.

In general, the controller 122 includes a processor 121, an actuator 123, and a signal input 125. The controller 122 also can include an actuator arrangement 123 for driving the skin piercing members 34 of each sensor module 20 between the extended and retracted positions to obtain a fluid sample. For example, the actuator arrangement 123 can be configured to push the skin piercing member anchor 30 in a distal direction relative to the analysis cell housing 28 and to pull the skin piercing member anchor 30 in a proximal direction relative to the analysis cell housing 28. The actuator arrangement 123 can be mechanically connected to the sensor module 20 by a rod, piston, or other type of mechanical connector 126. Alternatively, the actuator arrangement can provide movement instructions to the sensor module 20 via an electrical connection.

The processor 121 instructs the actuator arrangement 123 when to operate the sensor module 20 to obtain a fluid sample for analysis. The processor 121 also can instruct the module holder 124 and/or the actuator arrangement 123 to eject the used sensor module 20. In certain embodiments, the analyte monitoring system 120 also can include a drug/chemical delivery system. In such embodiments, the processor 121 also instructs the actuator arrangement 123 also initiates the delivery of a drug/chemical (e.g., insulin) from a drug/chemical reservoir 152 along a drug/chemical line 150 as instructed by the processor 121 as will be discussed in greater detail herein.

The signal input 125 receives the signal generated at the electrodes 100, 102 of the sensor module 20 after obtaining the fluid sample and provides the signal to the processor 121 for analysis. The processor 121 converts the data obtained from the signal to an analyte concentration level (e.g., a blood glucose reading) or other desired information. The processor 121 causes the display 127 to indicate the processed information to the user. Other information also can be presented on the display 127. In one embodiment, the display 127 is a visual display. In other embodiments, an audio display also can be used. Additional information can be provided to the processor 121 via a user interface 129 (e.g., buttons, switches, etc.).

The sensor module 20 can include structures that facilitate coupling the electrodes 100, 102 of the sensor module 20 to the signal input 123 of the controller 122. For example, as shown at FIG. 1, the sensor module 20 can include two contacts 106 and 108 that are electrically connected to the first and second electrodes 100, 102, respectively. The contacts 106, 108 include exposed outer portions protruding from the cell analysis housing 28 that can be electrically connected to the controller 122 by a wire, conductive tracing, or other type of electrical conductor 128. The contacts 106, 108 also include inner portions that extend within the module body 22 and make electrical contact with the respective electrodes 100, 102. The module body 22 can define contact receivers (e.g., receptacles, pads, slots, or other structures) for receiving and retaining the contacts 106, 108 within the module body 22.

The body 22 of the sensor module 20 also can have structures that facilitate mounting the module body 22 relative to one or more components of the overall analyte monitoring system 120. For example, embodiments of the analysis cell housing 28 can have an outer shape configured to fix the analysis cell housing 28 relative to the module holder 124. In such a fixed mounting configuration, the analysis cell housing 28 will remain at one location while the skin piercing member anchor 30 is driven distally along the axis 31 relative to the analysis cell housing 28 and pulled proximally along the axis 31 relative to the analysis cell housing 28. In the depicted embodiment, the analysis cell housing 28 includes a projection in the form of a tab 86 (FIGS. 4-6) that fits within a corresponding or mating receptacle provided in the module holder 124 and to which the module body 22 can be mounted as a subcomponent.

Similarly, the exterior of embodiments of the skin piercing member anchor 30 can include notches, shoulders, slots, or other structures that facilitate coupling the skin piercing member anchor 30 to the actuator arrangement 123. In the embodiment depicted in FIGS.1-6, the skin piercing member anchor 30 includes a hold feature 84 adapted to couple with a corresponding clip or latch provided on the actuator arrangement 123. The skin piercing member anchor 30 also can include tabs, slots, rails, or other structures that assist in guiding the axial movement of the skin piercing member anchor 30 along the axis 31 when the module body 22 is installed within the module holder 124. For example, an extension 86 of the piercing member anchor 30 can be adapted to slide within a corresponding slot provided in a portion of the module holder 124 in which the module body 22 is mounted (see FIGS. 3-6).

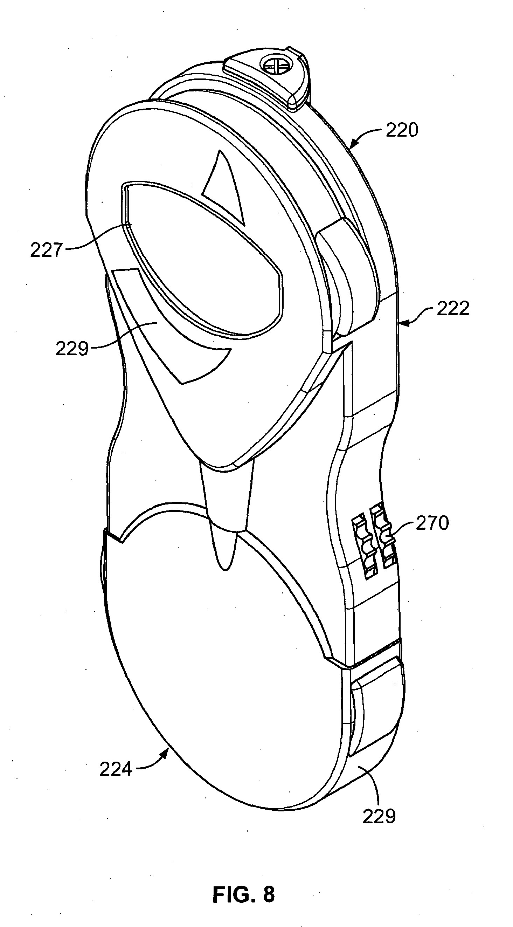

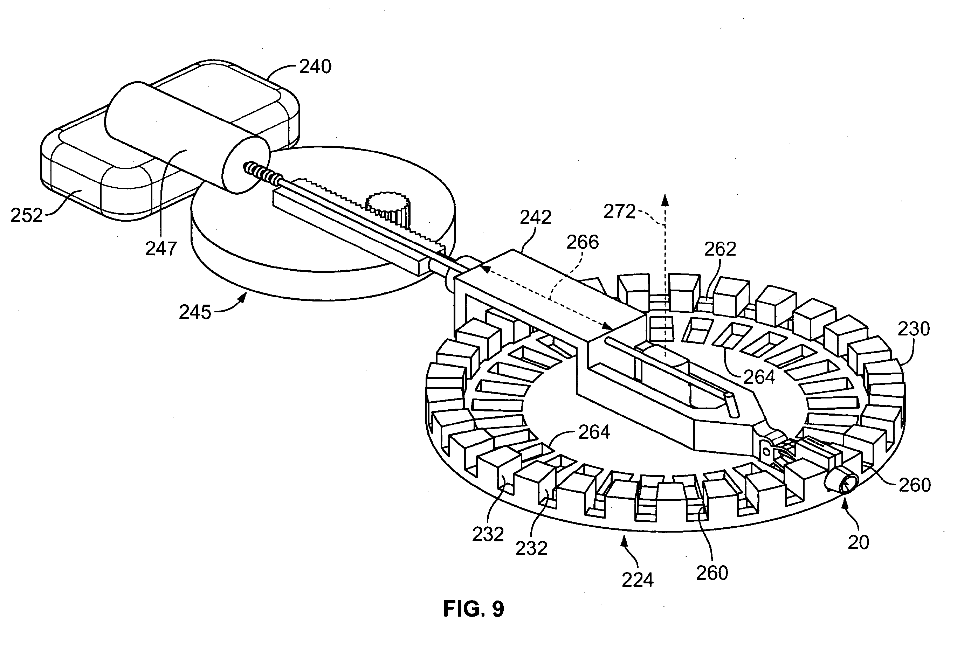

FIG. 8 shows one example embodiment of an analyte monitoring system 220. Generally, the analyte monitoring system 220 includes a base housing 222 holding metering electronics configured to measure analyte concentrations, such as a glucometer. The analyte monitoring system 220 also includes a disposable cartridge 224 in which a plurality of sensor modules 20 can be mounted. The cartridge 224 is of sufficient size that it can be readily handled by a diabetic patient. However, since the sensor modules 20 are housed within the cartridge 224, the sensor modules 20 can be manufactured at smaller sizes since they never need to be handled individually by the diabetic patient. In one embodiment, the cartridge 224 has a diameter less than about 5 centimeters (2 inches), whereas the bodies 22 of the sensor modules have lengths less than about 1.3 centimetres (0.5 inches) measured along the axis 31 when the module body 22 is in the extended orientation of FIG. 4. FIG. 9 shows a relative size between the individual sensor systems 20 and an outer housing 229 of the cartridge 224.

Referring to FIG. 9, the cartridge 224 is shown with the outer housing 229 removed to show a rotatable carrier wheel 230 that mounts within the outer housing 229 of the cartridge 224. The carrier wheel 230 is circular in shape and includes a plurality of sensor mounting stations 232 spaced about the outer circumference of the carrier wheel 230. Each of the sensor mounting stations 232 is adapted to receive one of the sensor modules 20. Preferably, the carrier wheel 230 is configured to hold at least ten of the sensor modules 20. More preferably, the carrier wheel 230 is configured to hold at least twenty of the sensor modules 20. Still more preferably, the carrier wheel 230 is configured to hold at least thirty of the sensor modules 20. In other embodiments, however, the carrier wheel 230 can be configured to hold a greater or fewer number of sensor modules 20.

Referring still to FIG. 9, when the sensor modules 20 are mounted at the sensor mounting stations 232, the axis 31 of each sensor module 20 is preferably aligned to extend radially outwardly from a center of the carrier wheel 230. Thus, the axis 31 of each sensor module 20 mounted to the wheel 230 is arranged in a radially spoked configuration.

Referring still to FIG. 9, the analyte monitoring system 220 also can include an actuator arrangement 240 for driving the skin piercing members 34 of the sensor modules 20 between the extended and retracted positions. As shown at FIG. 9, the actuator arrangement 240 includes an actuator linkage arm 242 that projects outside the permanent housing 222 and into the disposable cartridge 224. In certain embodiments, the outer housing 229 of the disposable cartridge 224 can have a two-piece configuration such that the housing pieces (e.g., front and back pieces) can be open to allow insertion of the linkage arm 242 into the cartridge 224. Alternatively, in certain embodiments, the outer housing 229 can be provided with an opening at its outer circumference and the wheel 230 can be provided with a gap or spacing between sensor mounting locations sufficiently large for the linkage arm 242 to be inserted through the opening in the housing 229 and through the spacing at the outer circumference of the wheel 230 and into the interior of the wheel 230.

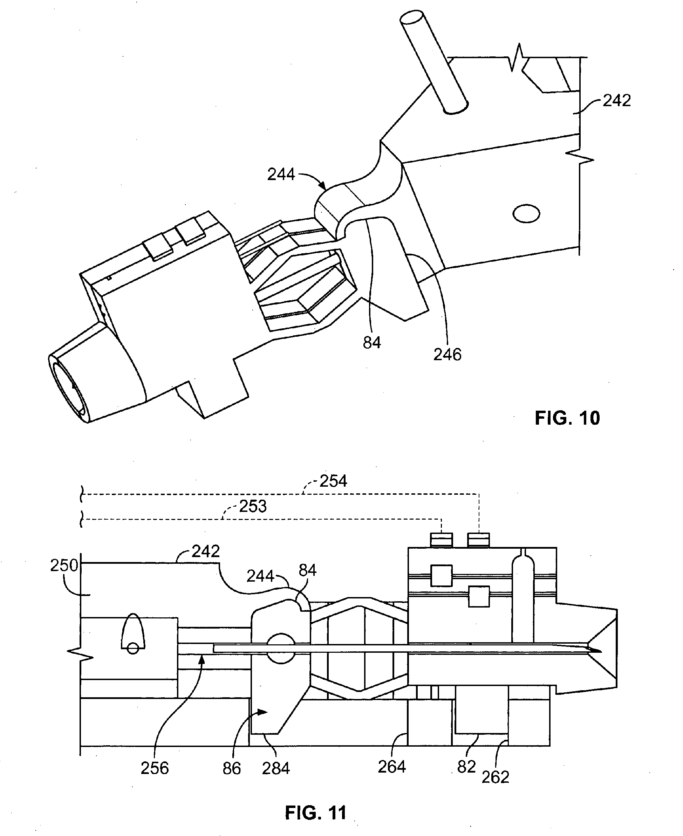

As shown at FIGS. 10 and 11, the linkage arm 242 includes a latch 244 that engages the holding element 84 of the sensor module 20 to couple the linkage arm 242 to the skin piercing member anchor 30 of the sensor module 20. The latch 244 preferably has a flexible configuration that facilitates connecting and disconnecting the latch 244 with the holding elements 84 of the sensor modules 20 supported by the carrier wheel 230. The linkage arm 242 also can include a drug/chemical (e.g., insulin) line connector 256 that connects to the proximal end of the skin piercing member 34 (shown as a canulla). The drug/chemical line connector 256 provides fluid communication between the interior of the skin piercing member 34 and an insulin line 250 routed to an insulin reservoir 252. As shown at FIG. 11, the linkage arm 242 also includes electrical conductors 253, 254 that respectively make contact with the electrical contacts 106, 108 of the sensor module 20 so that the electrodes 100, 102 can be electrically connected to the processor (e.g., glucometer) when the cartridge 224 is loaded into the intermediate housing 222.

Referring to FIGS. 9 and 11, each of the sensor mounting locations 232 defined by the carrier wheel 230 includes a slot 260 for receiving the sensor modules 20 with the distal tip 72 of each sensor module body 22 projecting outwardly beyond the outer circumference of the wheel 230. Each sensor mounting locations 232 also includes a pocket 262 positioned radially inwardly from the slot 260 for receiving the mounting projection 82 of the module body 22. With the mounting projection 82 located within the pocket 262, interference between the pocket 262 and the mounting projection 82 prevents radial movement of the module body 22 relative to the carrier wheel 230. Each of the sensor mounting positions 232 also includes a radial slot 264 positioned radially inwardly from the pocket 262. The slot 264 is sized to receive the extension 86 from the skin piercing member anchor 30. The slot 264 functions to guide the skin piercing member anchor 30 along a straight radial path as the skin piercing member anchor 30 is driven radially to move the skin piercing member 34 between the extended and retracted positions.

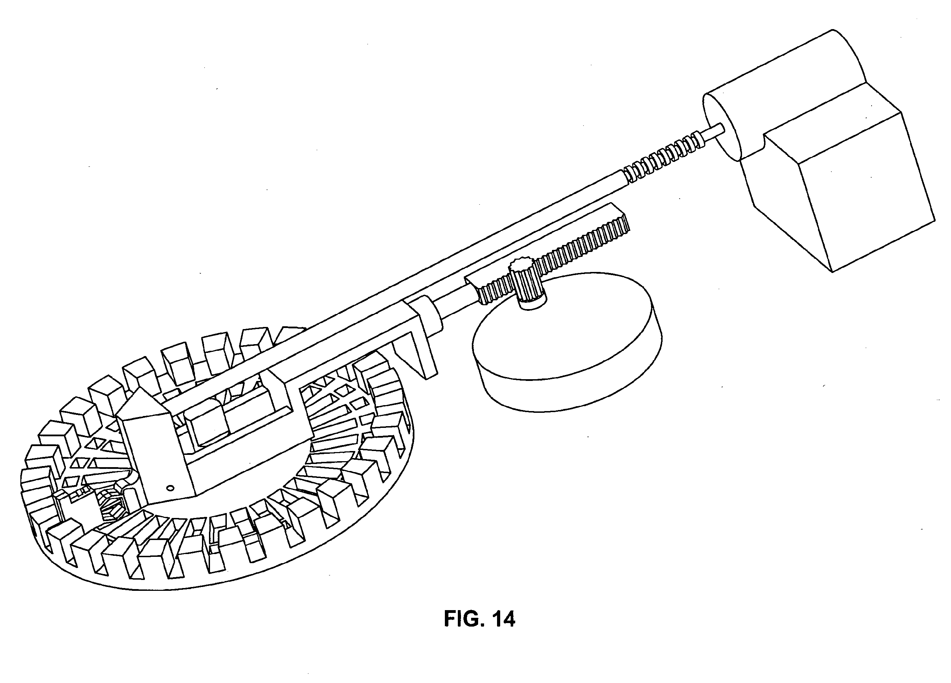

Referring still to FIG. 9, the analyte monitoring system 220 also includes a drive mechanism (e.g., a stepper motor with a rack and pinion gear) 245 located within the intermediate housing 222. The rack of the rack and pinion gear is fixably connected to linkage arm 242. In this way, the drive mechanism 245 can be used to move the linkage arm 242 relative to the carrier wheel 230 along an axis 266 that is coaxial with the axis 31 of the sensor module 20 to which the linkage arm 242 is coupled. The intermediate housing 222 also can house a drug reservoir 252 (e.g., insulin reservoir) and a pump (e.g., a micro pump) 247 for pumping the drug from the reservoir 252 through the drug line 250 to the skin piercing member 34 of the sensor module 20 to which the linkage arm 242 is coupled. FIG. 14 is a perspective view of another analyte monitoring system showing how the sensor module of FIG. 1 can be arranged within a cartridge wheel of the system in accordance with the principles of the present disclosure.

Referring back to FIG. 8, the intermediate housing 222 also includes an indexing wheel 270 that mechanically connects to the carrier wheel 230 when the disposable cartridge 224 is connected to the intermediate housing 222. By turning the indexing wheels 270, the carrier wheel 230 can be turned about its central axis 272 to index the wheel 230 to align a new sensor module 20 with the linkage arm 242. For example, in use, after a blood test has been conducted with one of the sensor modules 20 held by the carrier wheel 230, the linkage arm 242 disconnects from the spent sensor module 20. Thereafter, the user turns the indexing wheel 270 with their thumb to move the spent sensor module 20 out of alignment with the linkage arm 242 and to place the next, unused sensor module (not shown) in alignment with the linkage arm 242.

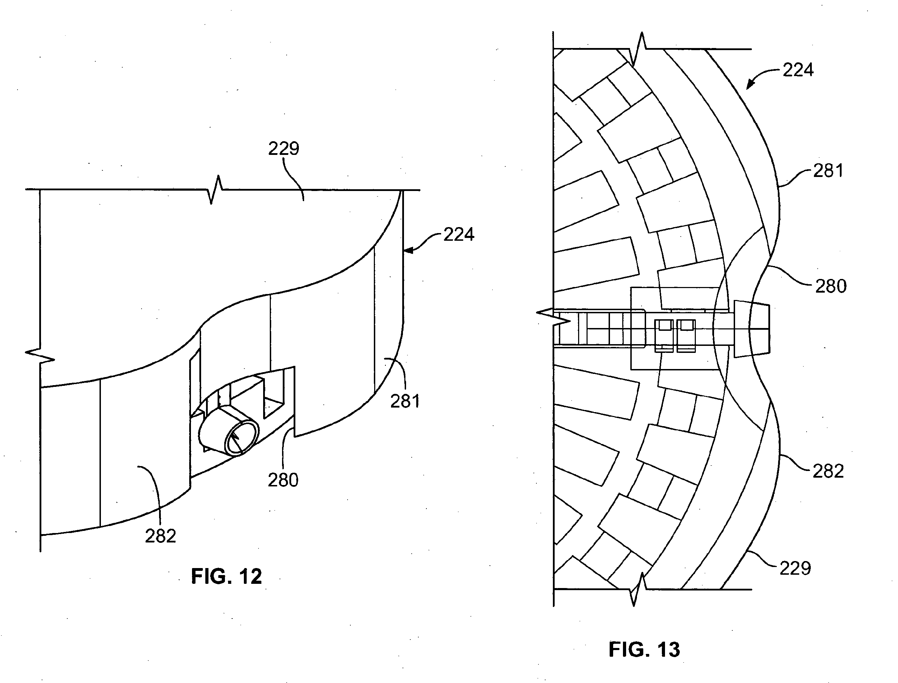

As shown at FIGS. 12 and 13, the outer case 229 of the disposable cartridge 224 includes a circumferential wall 281 that defines an interface port (e.g., an opening) 280 through which the distal tip 72 of the sensor module 20 being used projects. The wall 281 defines curved surfaces 282 on opposite sides of the interface port 280. During use of the analyte monitoring system 220, preferably the distal tip 72 and the curved surfaces 182 engage the patient's skin at an appropriate interface site (e.g., the patient's thigh, stomach, hand, etc.). The surfaces 282 can have a high tack texture so as to assist in spreading the patient's tissue to allow for improved capillary flow during fluid (e.g., blood) sampling.

In use, the user first removes the disposable cartridge 224 from its packaging and opens a meter access door of the housing 222 to load the cartridge 224 into a receiver bay of the housing 222. The meter electronics contained within the housing 222 determine whether that the cartridge 224 is properly loaded and whether the first sensor module 20 is appropriately positioned and ready for use. One of the sensor modules 20 is initially indexed into alignment with the linkage arm 242, which is arranged in a retracted orientation in which the latch 244 and the drug/chemical line connector 256 are positioned radially inwardly from the skin piercing member anchor 30 of the sensor system 220.

When a fluid testing sequence is initiated, the patient positions the curved surfaces 282 of the housing 222 at an appropriate sampling location on the user's skin and pushes the curved surfaces 282 of the housing 222 firmly against the tissue at the skin interface. The user also interacts with a user interface structure (e.g., presses a button) on the housing 222 to begin an analysis/dose cycle. Preferably, the analysis/dose cycle includes two insertion and retraction actuations of the piercing member 34 of the aligned sensor module 20 to different prescribed depths within the tissue. In certain embodiments, the system optionally performs a self analysis before initiating the cycle. In one such embodiment, the user must toggle the user interface a second time to initiate the cycle.

During the first insertion and retraction actuation of the cycle, the drive mechanism 245 of the analyte monitoring system 220 rapidly pushes the linkage arm 242 radially outwardly toward the skin piercing member anchor 30 of the sensor module 20 in alignment with the linkage arm 242. As the linkage arm 242 is driven radially outwardly, the latch 244 engages a tapered portion of the holding element 84 and flexes up and over the holding element 84 to a connected orientation (see FIG. 10). Concurrently, the drug line connector 256 connects to the proximal end of the skin piercing member 34.

As the linkage member 242 continues to be driven radially outwardly, a pushing surface 246 of the linking arm 242 engages a proximal end of the skin piercing member anchor 30 causing the skin piercing member anchor 30 and the skin piercing member 34 to be driven radially outwardly. As previously described, the hinge configuration of the flexible linkage 32 of the sensor module 20 allows the skin piercing member anchor 30 to be moved along the axis 31 relative to the analysis cell housing 28. The skin piercing member 34 slides through the guide portion 36c of the lumen 36 defined in the sensor module 20 to reach the skin interface site. The skin piercing member 34 is guided by the integral, living hinge-like feature 32 connecting the proximal and distal portions of the sensor module 20.

The skin piercing member 34 penetrates the patient's tissue sufficiently to reach a predetermined depth sufficient to reach a capillary blood field and is quickly retracted (first retraction) by the linkage member 242 back to the original position. Upon first retraction, the fluid sample is obtained at the tapered passage 36a and transported by capillary flow through the sample portion 36b of the passage 36. Such flow is facilitated by the air vent 90 defined by the analysis cell 50. The capillary slot 46 intercepts the axially flowing fluid sample and conveys the sample to the analysis cell 50.

An analysis of the fluid sample is performed at the two electrodes 100, 102 of the sensor module 20. A signal generated by the electrodes 100, 102 is conveyed through the contacts 106, 108 protruding from the sensor module 20 to conductors 253, 254 terminating at the meter electronics within the housing 222. The metering electronics determine an analyte (e.g., glucose) concentration level of the fluid sample. The metering electronics also can report the concentration level to the user via the display 227.

The metering electronics also can calculate an appropriate quantity of drug/chemical (e.g., insulin) to inject into the user based on the determined concentration level. Data indicating the calculated quantity is sent to drive mechanism 245. The drive mechanism 245 initiates the second rapid injection through the patient interface port 280 to a second predetermined depth in the tissue. When the piercing member 34 reaches the second depth, a pumping mechanism 247 delivers the prescribed unit volume of drug/chemical (e.g., the appropriate amount of insulin required to maintain the patient's glucose values based on the blood analysis).

The drive mechanism 245 radially retracts the linkage arm 242 beyond the original starting point of the cycle. As shown in FIG. 11, the drive mechanism 245 retracts the linkage arm 242 until the extension 86 of the skin piercing member anchor 30 engages a stop surface 284 of the guide slot 264 defined by the carrier wheel 230. Contact between the stop surface 284 and the extension 86 stops movement of the skin piercing member anchor 30 while the linkage member 242 continues to retract. As the linkage member 242 continues to retract, the latch 244 flexes up and over the holding element 84, thereby causing the linking arm 242 to be mechanically disconnected from the sensor module 20.

When the linkage arm 242 disengages from the sensor module 20, the metering electronics report the completion of the dose administration to the patient (e.g., via the display 227). To complete the cycle, the cartridge 224 is caused to rotate (e.g., automatically, manually, etc.) to move a new sensor module 20 into position. Sequential fluid sample readings and drug/chemical doses are repeated as required by the patient until the cartridge 224 completes a full rotation and is spent, at which point a new cartridge is loaded and the spent cartridge disposed. In certain embodiments, on board firmware controls all system functions, performs failsafe monitoring preventing reuse of sensors or spent cartridges or out of spec actuations and stores the data for each sensor and cartridge for telemetry to external data processing points.

## Claims

1. A sensor module (20) comprising:
an analysis cell housing (28) defining an analysis cell (50) and a passageway (36) providing access to the analysis cell (50) from a sample port of the analysis cell housing (28);
a member anchor (30) configured to move relative to the analysis cell housing (28) along an axis (31) that passes through the sample port;
a piercing member (34) securely coupled to the member anchor (30), the piercing member (34) being configured to slide along the axis (31) within at least a portion of the analysis cell housing (28) from a retracted position to an extended position when the member anchor (30) is moved relative to the analysis cell housing (28); and
an electrode arrangement arranged at least partially within the analysis cell housing, the electrode arrangement being arranged in fluid communication with the analysis cell, the electrode arrangement including a wire, or a fiber (100, 102).

2. The sensor module (20) of claim 1, wherein a flexible linkage (32) mechanically connects the member anchor (30) to analysis cell housing (28).

3. The sensor module (20) of claim 2, wherein the flexible linkage (32) includes two linkage members (32a, 32b) that each include an intermediate flex or hinge point (70) that enables the linkage members (32a, 32b) to flex radially outwardly relative to the axis when the member anchor (30) is moved in a distal direction relative to the analysis cell housing (28) and to flex inwardly when the anchor member (30) is moved in a proximal direction relative to the analysis cell housing (28).

4. The sensor module (20) of claim 1, wherein the fiber (100, 102) has a dielectric core surrounded by a conductive layer, which is covered with a sensing layer including a redox enzyme.

5. The sensor module (20) of claim 4, wherein the redox enzyme is glucose oxidase or glucose dehydrogenase.

6. The sensor module (20) of claim 1, wherein working, reference, and counter electrodes are provided in fluid communication with the analysis cell (50).

7. The sensor module (20) of claim 1, wherein the passage (36) has a tapered portion (36a) through which a blood sample enters the analysis cell housing (28).

8. The sensor module (20) of claim 7, wherein the passage (36) includes a capillary slot (46) that provides a larger cross-section to provide a capillary space along the piercing member (34) for allowing the blood sample to travel by capillary action from the tapered portion (36a) of the passage (36) to the analysis cell (50).

9. The sensor module (20) of claim 1, wherein the piercing member (34) includes a cannula, a needle, or a lancet.

10. The sensor module (20) of claim 1, wherein a drug/chemical line connector (256) provides fluid communication between the interior of the piercing member (34) and an insulin line (250) routed to an insulin reservoir (252).

11. The sensor module (20) of claim 1, wherein the analysis cell (50) has a vent (90) that allows air within the analysis cell (50) to be displaced by a blood sample.

12. The sensor module (20) of claim 1, wherein the analysis cell (50) is elongated in a direction that is generally perpendicular relative to the axis (31).

13. The sensor module (20) of claim 1, wherein a sample transport portion (36b) has a larger transverse cross-sectional area than a piercing member guide portion (36c).

14. The sensor module (20) of claim 1, wherein a distal tip (72) of the analysis cell housing (28) includes a circular skin engaging surface (74).

## Patentansprüche

1. Sensormodul (20) umfassend:
ein Analysezellengehäuse (28), das eine Analysezelle (50) und einen Durchgang (36) definiert, der von einem Probenzugang des Analysezellengehäuses (28) aus einen Zugriff auf die Analysezelle (50) ermöglicht;
einen Elementanker (30), der sich relativ zu dem Analysezellengehäuse (28) entlang einer Achse (31) bewegen kann, die durch den Probenzugang hindurch verläuft;
ein Stechelement (34), das fest mit dem Elementanker (30) verbunden ist, wobei das Stechelement (34) derart ausgebildet ist, dass es innerhalb wenigstens eines Teils des Analysezellengehäuses (28) entlang der Achse (31) von einer zurückgezogenen Position in eine ausgefahrene Position gleiten kann, wenn der Elementanker (30) relativ zu dem Analysezellengehäuse (28) bewegt wird; und
eine Elektrodenanordnung, die wenigstens teilweise innerhalb des Analysezellengehäuses angeordnet ist, wobei die Elektrodenanordnung in Fluidverbindung mit der Analysezelle steht, wobei die Elektrodenanordnung einen Draht oder eine Faser (100, 102) beinhaltet.

2. Sensormodul (20) nach Anspruch 1, wobei eine flexible Verbindung (32) den Elementanker (30) mechanisch mit dem Analysezellengehäuse (28) verbindet.

3. Sensormodul (20) nach Anspruch 2, wobei die flexible Verbindung (32) zwei Verbindungselemente (32a, 32b) beinhaltet, von denen jedes einen Zwischenbiege- oder -gelenkpunkt (70) aufweist, der es den Verbindungselementen (32a, 32b) ermöglicht, sich relativ zu der Achse radial nach außen zu biegen, wenn der Elementanker (30) relativ zu dem Analysezellengehäuse (28) in einer distalen Richtung bewegt wird, und sich nach innen zu biegen, wenn der Elementanker (30) relativ zu dem Analysezellengehäuse (28) in einer proximalen Richtung bewegt wird.

4. Sensormodul (20) nach Anspruch 1, wobei die Faser (100, 102) einen dielektrischen Kern aufweist, der von einer leitenden Schicht umgeben ist, welche mit einer fühlenden Schicht bedeckt ist, die ein Redoxenzym beinhaltet.

5. Sensormodul (20) nach Anspruch 4, wobei das Redoxenzym Glukose-Oxidase oder Glukose-Dehydrogenase ist.

6. Sensormodul (20) nach Anspruch 1, wobei Arbeits-, Bezugs- und Gegenelektroden in Fluidverbindung mit der Analysezelle (50) vorgesehen sind.

7. Sensormodul (20) nach Anspruch 1, wobei der Durchgang (36) einen konischen Bereich (36a) aufweist, durch welchen eine Blutprobe in das Analysezellengehäuse (28) gelangt.

8. Sensormodul (20) nach Anspruch 7, wobei der Durchgang (36) eine Kapillaraussparung (46) aufweist, die mit einem größeren Querschnitt versehen ist, um entlang des Stechelements (34) einen Kapillarraum zur Verfügung zu stellen, der es der Blutprobe erlaubt, aufgrund von Kapillarwirkung von dem konischen Bereich (36a) des Durchgangs (36) zu der Analysezelle (50) zu wandern.

9. Sensormodul (20) nach Anspruch 1, wobei das Stechelement (34) eine Kanüle, eine Nadel oder eine Lanzette ist.

10. Sensormodul (20) nach Anspruch 1, wobei ein Arzneimittel/Chemikalien-Leitungs-Verbinder (256) eine Fluidverbindung zwischen dem Inneren des Stechelements (34) und einer Insulinleitung (250) herstellt, die mit einem Insulinreservoir (252) verbunden ist.

11. Sensormodul (20) nach Anspruch 1, wobei die Analysezelle (50) eine Entlüftungsöffnung (90) aufweist, welche es erlaubt, dass Luft innerhalb der Analysezelle (50) von einer Blutprobe verdrängt wird.

12. Sensormodul (20) nach Anspruch 1, wobei die Analysezelle (50) eine Längserstreckung in einer Richtung aufweist, die im Wesentlichen senkrecht zu der Achse (31) verläuft.

13. Sensormodul (20) nach Anspruch 1, wobei ein Probentransportbereich (36b) eine größere in Querrichtung verlaufende Querschnittsfläche aufweist als ein Stechelementführungsbereich (36c).

14. Sensormodul (20) nach Anspruch 1, wobei eine distale Spitze (72) des Analysezellengehäuses (28) eine kreisförmige Hautanlagefläche (74) aufweist.

## Revendications

1. Module de capteur (20) comprenant :
un boîtier de cellule d'analyse (28) définissant une cellule d'analyse (50) et un passage (36) permettant d'accéder à la cellule d'analyse (50) depuis un orifice pour échantillons du boîtier de cellule d'analyse (28) ;
un ancrage d'élément(30) configuré pour se déplacer par rapport au boîtier de cellule d'analyse (28) le long d'un axe (31) qui traverse l'orifice pour échantillons ;
un élément de perçage (34) solidement couplé à l'ancrage d'élément (30), l'élément de perçage (34) étant configuré pour coulisser le long de l'axe (31) dans au moins une partie du boîtier de cellule d'analyse (28) d'une position rétractée vers une position déployée lorsque l'ancrage d'élément (30) est déplacé par rapport au boîtier de cellule d'analyse (28) ; et
un agencement d'électrode agencé au moins partiellement dans le boîtier de cellule d'analyse, l'agencement d'électrode étant agencé en communication fluidique avec la cellule d'analyse, l'agencement d'électrode incluant un fil métallique, ou une fibre (100, 102).

2. Module de capteur (20) selon la revendication 1, dans lequel une liaison flexible (32) raccorde mécaniquement l'ancrage d'élément (30) au boîtier de cellule d'analyse (28).

3. Module de capteur (20) selon la revendication 2, dans lequel la liaison flexible (32) inclut deux éléments de liaison (32a, 32b) qui incluent chacun un point d'inflexion ou d'articulation intermédiaire (70) qui permet aux éléments de liaison (32a, 32b) de fléchir radialement vers l'extérieur par rapport à l'axe lorsque l'ancrage d'élément (30) est déplacé dans une direction distale par rapport au boîtier de cellule d'analyse (28) et de fléchir vers l'intérieur lorsque l'ancrage d'élément (30) est déplacé dans une direction proximale par rapport au boîtier de cellule d'analyse (28).

4. Module de capteur (20) selon la revendication 1, dans lequel la fibre (100, 102) possède un noyau diélectrique entourée d'une couche conductrice, qui est recouverte d'une couche de détection incluant une enzyme oxydo-réductase.

5. Module de capteur (20) selon la revendication 4, dans lequel l'enzyme oxydo-réductase est la glucose oxydase ou la glucose déshydrogénase.

6. Module de capteur (20) selon la revendication 1, dans lequel les électrodes de travail, de référence et contre-électrodes sont disposées en communication fluidique avec la cellule d'analyse (50).

7. Module de capteur (20) selon la revendication 1, dans lequel le passage (36) comporte une partie inclinée (36a) par laquelle un échantillon de sang pénètre dans le boîtier de cellule d'analyse (28).

8. Module de capteur (20) selon la revendication 7, dans lequel le passage (36) inclut une fente capillaire (46) qui prévoit une section transversale plus importante pour obtenir un espace capillaire le long de l'élément de perçage (34) permettant ainsi à l'échantillon de sang de se déplacer par action capillaire depuis la partie inclinée (36a) du passage (36) à la cellule d'analyse (50).

9. Module de capteur (20) selon la revendication 1, dans lequel l'élément de perçage (34) inclut une canule, une aiguille ou une lancette.

10. Module de capteur (20) selon la revendication 1, dans lequel un connecteur de voie de médicament/substance chimique (256) permet la communication fluidique entre l'intérieur de l'élément de perçage (34) et une voie d'insuline (250) acheminée vers un réservoir d'insuline (252).

11. Module de capteur (20) selon la revendication 1, dans lequel la cellule d'analyse (50) comporte un évent (90) qui permet à l'air présent dans la cellule d'analyse (50) d'être déplacé par un échantillon de sang.

12. Module de capteur (20) selon la revendication 1, dans lequel la cellule d'analyse (50) est allongée dans une direction qui est généralement perpendiculaire à l'axe (31).

13. Module de capteur (20) selon la revendication 1, dans lequel une partie de transport d'échantillon (36b) présente une section transversale plus importante qu'une partie de guidage de l'élément de perçage (36c).

14. Module de capteur (20) selon la revendication 1, dans lequel une extrémité distale (72) du boîtier de cellule d'analyse (28) inclut une surface d'engagement de la peau circulaire (74).
